**Europäisches Patentamt**

**European Patent Office**

**Office européen des brevets**

(11) Veröffentlichungsnummer: **0 475 541 A1**

# (12) EUROPÄISCHE PATENTANMELDUNG

(21) Anmeldenummer: **91250197.0**

(22) Anmeldetag: **18.07.91**

(51) Int. Cl.5: **C02F 3/10, C12N 11/04, C02F 3/06, C02F 3/34**

(30) Priorität: **24.08.90 DE 4027223**

(43) Veröffentlichungstag der Anmeldung: **18.03.92 Patentblatt 92/12**

(84) Benannte Vertragsstaaten: **DE DK FR GB IT NL**

(71) Anmelder: **Preussag Noell Wassertechnik GmbH**
**Alfred-Nobel-Strasse 20**
**W-8700 Würzburg 1(DE)**

(72) Erfinder: **Wildenauer, Franz Xaver, Dr.**
**Dorfstrasse 1b**
**W-2861 Ritterhude(DE)**
Erfinder: **Menzel, Roman, Dr.**
**Ouellenweg 10**
**W-4450 Lingen(DE)**
Erfinder: **Vetter, Helmut**
**Oslebshauser Heerstrasse 156**
**W-2800 Bremen 21(DE)**
Erfinder: **Vorlop, Klaus-Dieter, Dr.**
**Hochstrasse 7**
**W-3300 Braunschweig(DE)**
Erfinder: **Remmers, Peter**
**Lessingplatz 6**
**W-3300 Braunschweig(DE)**

(74) Vertreter: **Kaiser, Henning**
**SALZGITTER AG Patente und Lizenzen**
**Kurfürstendamm 32 Postfach 15 06 27**
**W-1000 Berlin 15(DE)**

(54) **Verfahren zum biologischen Abbau persistenter organischer Stoffe.**

(57) Zum biologischen Abbau persistenter organischer Stoffe wie Pestizide, halogenierte, aliphatische Kohlenwasserstoffe usw. in Wasser werden polymerfixierte Mikroorganismen, die als Biokatalysatoren in Gelkugeln eingeschlossen sind, verwendet. Die Gelkugeln werden durch Eintropfen einer Polyvinylalkohol-Lösung in z. B. flüssigen Stickstoff und sehr langsames Auftauen hergestellt. Hierdurch werden äußerst stabile und elastische Körper gebildet. In den Gelkugeln können je nach dem abzubauenden Stoff Adsorptionsmittel und Lösungsvermittler zugesetzt werden. Die Behandlung des Wassers erfolgt in einem oder mehreren Reaktoren, in denen eine intensive Vermischung der kugelförmigen Biokatalysatoren mit dem Wasser durchgeführt wird.

Die Erfindung betrifft ein Verfahren zum biologischen Abbau persistenter organischer Stoffe, die in Wasser enthalten sind. Der Abbau erfolgt in Reaktoren mittels in Gelkugeln polymerfixierten Biokatalysatoren. Das Wasser kann Abwasser aus industriellen oder kommunalen Kläranlagen, von chemischen Prozessen, Oberflächenwasser oder kontaminiertes Grundwasser sein.

Die Anwendung von Biokatalysatoren mit in Gelkugeln immobilisierten Mikroorganismen wurde schon verschiedentlich vorgeschlagen und labormäßig durchgeführt. Gelkugeln aus Polymeren biologischer Herkunft, wie beispielsweise Alginat oder Carrageenan werden bei Scherkräften, wie sie in technischen Reaktoren vorkommen, mechanisch zerrieben. Außerdem werden sie durch in Trink- und Abwasser vorkommende Mikroorganismen angegriffen und rasch zersetzt. Ca-Alginat löst sich außerdem in Gegenwart von in geringer Konzentration im Abwasser vorkommenden Phosphat durch Ausfällung von Calcium-Phosphat auf.

Als ein sich im Abwasser inert verhaltendes synthetisches Material wurden schon Polyurethane vorgeschlagen. Die Ausgangsstoffe dieses Materials sind jedoch hochgiftig, so daß sie die einzuschließenden Mikroorganismen noch während der Polymerisation abtöten oder wenigstens reduzieren. Polyurethane haben darüberhinaus eine geringe Durchlässigkeit für gelöste organische Substanzen und besitzen als weiteren Nachteil, daß sich im Inneren eines Polyurethankatalysators während der Polymerisation kaum Hohlräume ausbilden, so daß die eingeschlossenen Mikroorganismen keinen Raum zum weiteren Wachstum haben.

Der Erfindung liegt die Aufgabe zugrunde, ein wirtschaftlich einsetzbares Verfahren zum biologischen Abbau persistenter organischer Stoffe in Wasser mittels Biokatalysatoren zu schaffen, die aus in Gelkugeln immobilisierten, polymerfixierten Mikroorganismen bestehen und gegen auftretende mechanische und chemische Beanspruchungen für lange Zeit beständig sind.

Die Lösung dieser Aufgabe ergibt sich aus Anspruch 1. Die Unteransprüche betreffen spezielle Ausführungen für bestimmte Kontaminationen und weitere Ausgestaltungen des Verfahrens.

Es wurde gefunden, daß aus einer wässrigen Lösung eines Polyvinylalkohols mit einem Polymerisierungsgrad über 1200 und einem Hydrolysegrad von mindestens 98% mit einem Verhältnis von 40 bis 150 g Polyvinylalkohol, vorzugsweise 70 bis 100 g zu 1 l Wasser eine hochviskose Lösung hergestellt werden kann, daß aus dieser Lösung durch Eintropfen in eine tiefkalte Flüssigkeit von unter -30° C, vorzugsweise in flüssigen Stickstoff kugelförmige Körper hergestellt werden können und daß aus diesen gefrorenen Körpern bei langsamem Auftauen, insbesondere im Temperaturbereich zwischen -20° C und 5° C mit einer Erwärmungsgeschwindigkeit von nicht mehr als 20° C/h, vorzugsweise bis etwa 4° C/h zwischen -5 und +5° C stabile und elastische Gelkugeln entstehen, in denen die der Lösung zugegebenen Mikroorganismen oder ähnliche als Biokatalysator geeignete Stoffe im Polymer fixiert sind. Der hochviskosen Polyvinylalkohol-Lösung kann eine weichmachende, die Elastizität der hergestellten Gelkugeln verbessernde Komponente, wie Glyzerin oder Zucker bis zu 40 Gew.-% zugefügt werden. Als weichmachende Zusätze kommen auch wasserlösliche organische Verbindungen mit Hydroxylgruppen, wie z. B. Ethylenglycol in Frage. Die Zusätze können gleichzeitig als Nährstoffquelle für lebende Mikroorganismen dienen.

Die hergestellten Gelkugeln besitzen eine glatte abriebfeste Oberfläche und sind elastisch deformierbar. Sie werden durch in Reaktionsbehältern auftretende Scherkräfte sowie durch in dem zu behandelnden Wasser enthaltenen chemischen Bestandteile nicht zerstört.

Für den biologischen Abbau persistenter organischer Stoffe werden der Polyvinylalkohol-Lösung neben geeigneten Mikroorganismen mit Wasser nicht mischbare Lösungsvermittler und/oder feste oder flüssige Adsorptionsmittel zugefügt, die in den Gelkugeln eingeschlossen bleiben.

Für den Abbau von polyzyklischen Aromaten (sogenannten PAK's) wird als in Tropfenform in den Gelkugeln eingeschlossener flüssiger Lösungsvermittler die Verbindung 2, 2, 4, 4, 6, 8, 8 Heptamethylnonan bevorzugt. In diesem Lösungsvermittler reichern sich PAK- und PCB-Verbindungen an und stehen so den Mikroorganismen für den Abbau zur Verfügung. Die zu lösenden und abzubauenden Verbindungen sind beispielsweise Naphthalin, Phenanthren, Fluoren, Chrysen, Benz(a)pyren, Benzo (g, h, i)-perylen.

Für den Abbau von Pestiziden ist als Adsorptionsmittel $\beta$-Cyclodextran zum Komplexieren dieser Stoffe geeignet. Sie werden in dem Adsorptionsmittel nicht nur wenigstens vorübergehend für den Abbau gespeichert, sondern wirken in den erreichten größeren Konzentrationen auch weniger giftig. Auf diese Weise abbaubare Pestizide sind beispielsweise Folpet, Capton und TMTD.

Für den Abbau von halogenierten, aliphatischen Kohlenwasserstoffen, wie Dichlorpropan, Trichlorpropan, Dichlorethan, Trichlorethan, Tetrachlorethylen und Methylchlorid werden dem Biokatalysator anorganische feste Adsorbentien, wie pulverisierte Aktivkohle oder Lewatit OC 1062 (ein Produkt der Fa. Bayer) zugemischt. Alternativ können bei dieser Anwendung auch einfache Lösungsvermittler, wie synthetische oder pflanzliche Öle zugegeben werden.

Zum Abbau von schwer abbaubaren, persistenten Verbindungen werden Mikroorganismen aus kontaminiertem Böden oder Abwasser entnommen und auf Selektivnährböden angereichert. Die auf diese Weise gewonnenen Mischkulturen enthalten bevorzugt Arten der Gattungen Pseudomonas, Acinetobacter und Xanthobacter sowie Pilze der Gattung Pleurotus. Die für den jeweiligen Anwendungsfall geeigneten Mischkulturen werden in einer Fermentationsanlage gezüchtet, vorzugsweise mittels Separatoren aufkonzentriert und der Polyvinylalkohol-Lösung in einer Konzentration von vorzugsweise etwa 1 Gew.-% beigemischt.

Es ist zweckmäßig, kontaminiertes Wasser oder Abwasser zur Vorbereitung der biologischen Behandlung physikalisch und/oder chemisch vorzubehandeln. Dabei wird zunächst in einem ersten Schritt durch Zugabe von Säure oder Lauge ein geeigneter pH-Wert des Wassers eingestellt. Anschließend werden durch anorganische Fällungsmittel, wie z. B. Aluminiumoxid und/oder Eisen-II-Sulfat organische ungelöste Verunreinigungen ausgefällt.

Wenn in dem Wasser Zwischenprodukte enthalten sind, die aus insbesondere der chargenweisen Synthese chemischer Verbindungen anfallen, werden die Verbindungen einer mikrobiellen Zersetzung durch den Biokatalysator zugänglich gemacht, indem das Wasser zuvor einer Naßoxidation unterzogen wird. Derartige chemische Verbindungen treten beispielsweise auf bei der Reduktion von aromatischen Nitroverbindungen zu Anilinen, bei der Sulfonierung und Sulfochlorierung von aromatischen Zwischenprodukten, bei der Nitrierung von Arylsulfonsäuren, bei Chlorierung von Arylsulfonsäuren, bei der Diazotierung und Herstellung von Azofarbstoffen.

Bei der Naßoxidation werden dem Wasser Eisenionen und Chinonbildner zudosiert. Es wird dann unter Druck erwärmt und in einem Blasensäulen-Reaktor mit aus dem Boden der Säule austretenden Sauerstoff behandelt. Vorzugsweise erfolgt die Naßoxidierung in der Blasensäule als Niederdruckoxidation bei 140° C ohne Überdruck, um die Betriebskosten niedrig zu halten. Eine Mittel- oder Hochdruckoxidation mit einer Temperatur bis 280° C und einem Druck bis 120 bar sind jedoch möglich.

Durch die Oxidation wird bei aromatische Verbindungen mit NO$_3$-, NH$_2$, -COOH und -SO$_3$H - Gruppen eine Ringspaltung und damit eine Verbesserung des biologischen Abbaus erreicht.

Flüchtige Verbindungen, wie halogenierte aliphatische Kohlenwasserstoffe werden zweckmäßigerweise aus dem Wasser angereichert, indem sie nach der Fällung mit Luft oder Dampf ausgestrippt und kondensiert werden, wobei dann das Kondensat kontinuierlich oder diskontinuierlich einem Reaktor mit den Biokatalysatoren zugeführt wird.

Als Reaktoren sind Behälter geeignet, die eine intensive Durchmischung der kugelförmigen Biokatalysatoren mit dem Wasser und einem Begasungsmedium ermöglichen, gleichzeitig jedoch hohe Scherkräfte vermeiden. Es kommen beispielsweise Reaktoren für 3-Phasen-Wirbelschicht mit internen oder externen Schlaufen und Blasensäulen-Reaktoren in Frage. Häufig genügt jedoch die Installation eines Düsenbodens oder eines Rührwerkes in einem Behälter für eine ausreichende Durchmischung. Eine Begasung mit reinem Sauerstoff und eine Erhöhung des Reaktorsdrucks zur Verbesserung der Sauerstoff-Löslichkeit ist von Vorteil, wenn in dem Wasser Verbindungen vorhanden sind, die durch intensive Begasung ausgetrieben werde können.

Es ist vorteilhaft, in dem Reaktor Bedingungen einzustellen, die das Wachstum der polymerfixierten Mikroorganismen und/oder deren Abbauaktivität fördern. Die Bedingungen sind den besonderen physiologischen Anforderungen der jeweiligen Spezies beziehungsweise Mischkultur anzupassen. In der Regel liegt eine geeignete Temperatur zwischen 15 und 55° C, wobei in vielen Fällen eine Temperatur von ca. 35° C zu bevorzugen ist. Eine geeignete Temperatur kann auch die ursprüngliche Temperatur des Abwassers sein. Der pH-Wert ist auf einen Wert zwischen 4 und 10 einzustellen, wobei ein pH-Wert von 7 in vielen Fällen bevorzugt wird. Es kann jedoch auch der ursprüngliche pH-Wert des Abwassers optimal sein. Das Redoxpotential sollte zwischen -500 und +300 mV liegen, wobei auch hier das sich im Abwasser von selbst einstellende Redoxpotential das Optimale sein kann. Die Konzentration von gelöstem Sauerstoff sollte, soweit erforderlich, zwischen 0,1 mg O$_2$/l bis 10 mg O$_2$/l liegen. Ein im Normalfall bevorzugter Wert ist etwa 1 mg O$_2$/l Abwasser.

In weiterer Ausgestaltung der Erfindung kann der biologische Abbau gegebenenfalls nach physikalischer oder chemischer Vorklärung in mehreren Reaktionsbehältern stattfinden, in denen die Biokatalysatoren mit dem Wasser in Kontakt gebracht werden. Bei der Verwendung mehrerer Reaktionsbehälter ist es möglich, die Bedingungen in verschiedenen Behältern unterschiedlich einzustellen und/oder Biokatalysatoren verschiedener Art einzusetzen. Es kann ferner zweckmäßig sein, in einem Behälter zusammen mit dem Wasser geeignete Substrate einzugeben, die das Wachstum der Mikroorganismen im Biokatalysator fördern oder die zusammen mit den Biokatalysatoren einen Abbau der persistenten organischen Stoffe durch einen Co-Metabolismus erst ermöglichen.

Bei der Verwendung mehrerer Reaktoren ist es möglich, in einem oder mehreren den biologischen Abbau gegebenenfalls unter weiterer Aktivierung der Biokatalysatoren durch Zugabe geeigneter Substrate durchzuführen, während in einem oder mehreren anderen Reaktoren eine Reinigung und Entkeimung erfolgt. In einem Reaktionsbehälter kann das Wachstum unerwünschter konkurrierender Mikroorganismen außerhalb des Biokatalysators unterdrückt oder wenigstens reduziert werden, indem das Medium umgepumpt und in geeigneter Weise z. B. durch UV-Entkeimung oder Ozonisierung weitgehend entkeimt wird. Eine Beseitigung von Fremdkeimen auf der Oberfläche der Biokatalysatoren durch UV-Bestrahlung kann ohne Schädigung der polymerfixierten Mikroorganismen dadurch ermöglicht werden, daß der wässrigen Lösung die Gelkugeln lichtundurchlässig machende Stoffe, wie Ruß oder pulverisierte Kohle zugesetzt werden.

Die in der eingangs beschriebenen Weise hergestellten Gelkugeln mit polymerfixierten Biokatalysatoren halten die Mikroorganismen in den Kugeln zurück, schützen sie gegen das Eindringen von Fremdkeimen und gegen eine Abweidung durch Protozoen oder andere höhere Organismen, was insbesondere bei einer niedrigen Substratkonzentration und dadurch bedingtem geringen Wachstum der Mikroorganismen vorteilhaft ist, sind aber durchlässig für die abzubauenden Verbindungen und die Stoffwechselprodukte der Mikroorganismen.

**Patentansprüche**

1. Verfahren zum biologischen Abbau persistenter organischer Stoffe, die in Wasser enthalten sind, in Reaktoren mittels polymerfixierter Mikroorganismen, die als Biokatalysatoren in Gelkugeln eingeschlossen sind, die aus einer wässrigen Lösung eines Polyvinylalkohols mit einem Hydrolysegrad von mindestens 98% und einem Verhältnis von 40 bis 150 g Polyvinylalkohol zu ein Liter Wasser durch Eintropfen dieser Lösung in eine wenigstens unter -30° C kalte Flüssigkeit und anschließendes Auftauen der gefrorenen Kugeln im Temperaturbereich von -20° C bis 5° C mit einer Erwärmungsrate von bis zu etwa 20° C/h hergestellt werden und die neben geeigneten Mikroorganismen mit Wasser nicht mischbare Lösungsvermittler und/oder feste oder flüssige Adsorptionsmittel enthalten.

2. Verfahren nach Anspruch 1 für den Abbau polyzyklischer Aromaten wie Naphthalin, Phenanthren, Fluren, Chrysen, Benz(a)pyren, Benzo-(g, h, i)-perylen, wobei als flüssiger Lösungsvermittler, in dem sich die Aromaten wenigstens vorübergehend bis zum Abbau durch die Mikroorganismen anreichern, Heptamethylnonan als tropfenförmige Einschlüsse in den Biokatalysator-Kugeln verwendet wird.

3. Verfahren nach Anspruch 1, insbesondere für den Abbau von Pestiziden, wie Folpet, Capton, TMTD, wobei in den Gelkugeln als Adsorptionsmittel β-Cyklodextran zum Komplexieren, Konzentrieren und vorübergehenden Speichen dieser Stoffe enthalten ist.

4. Verfahren nach Anspruch 1 für den Abbau von halogenierten aliphatischen Kohlenwasserstoffen, wobei Biokatalysatoren verwendet werden, deren Gelkugeln feste anorganische Adsorbentien, wie Aktivkohle enthalten.

5. Verfahren nach Anspruch 1 zum Abbau von halogenierten aliphatischen Kohlenwasserstoffen, wobei Biokatalysatoren verwendet werden, die Lösungsvermittler wie synthetische oder pflanzliche Öle enthalten.

6. Verfahren nach einem der Ansprüche 1 bis 5, dadurch gekennzeichnet, daß der Lösung zur Bildung der Gelkugeln Mikroorganismen zugesetzt werden, die aus mit den entsprechenden Schadstoffen kontaminiertem Boden oder Abwasser gewonnen und auf Selektivnährböden angereichert sind.

7. Verfahren nach Anspruch 6, dadurch gekennzeichnet, daß die Mikroorganismen Mischkulturen aus Arten der Gattungen Pseudomonas, Acinetobacter und Xanthobakter sowie Pilze der Gattung Pleurotus sind.

8. Verfahren nach Anspruch 6 oder 7, dadurch gekennzeichnet, daß geeignete Mikroorganismen in Fermentationsanlagen gezüchtet, mittels Separatoren aufkonzentriert und in einer Konzentration von etwa 1 Gew.-% der Lösung der Gelkugeln beigemischt werden.

9. Verfahren nach einem oder mehreren der vorangehenden Ansprüche, dadurch gekennzeichnet, daß in dem kontaminierten Wasser durch Zugabe von Säure oder Lauge ein geeigneter pH-Wert eingestellt wird und anschließend durch Zugabe von anorganischen Fällungsmitteln, wie Aluminiumoxid und/oder Eisen-II-Sulfat organische ungelöste und gelöste Verunreinigungen ausgefällt werden.

10. Verfahren nach einem oder mehreren der vorangehenden Ansprüche, dadurch gekennzeichnet, daß ein Wasser, welches aromatische Zwischenprodukte aus der chemischen Produktion enthält, einer Naßoxidation unterworfen wird, wobei dem Wasser Eisenionen und Chinonbildner zudosiert und in einem Blasensäulen-Reaktor bei erhöhter Temperatur Sauerstoff zugeführt wird, um eine Ringspaltung zur Verbesserung des biologischen Abbaus zu erreichen.

11. Verfahren nach einem oder mehreren der vorangehenden Ansprüche, dadurch gekennzeichnet, daß im Wasser enthaltene flüchtige Verbindungen wie halogenierte aliphatische Kohlenwasserstoffe mit Luft oder Dampf ausgestrippt und kondensiert werden und das Kondensat dem biologischen Abbau zugeführt wird.

12. Verfahren nach einem oder mehreren der vorangehenden Ansprüche, dadurch gekennzeichnet, daß in einem oder mehreren Reaktoren eine intensive Durchmischung der Biokatalysatoren mit dem Wasser und einem Begasungsmedium erfolgt, sowie Bedingungen für ein optimales Wachstum der polymerfixierten Mikroorganismen und deren Abbauaktivität durch Wahl einer den Mikroorganismen angepaßten Temperatur zwischen 15 und 55°C, eines pH-Wertes zwischen 4 und 10 sowie eines Redoxpotentials von -500 bis +300 mV geschaffen werden.

13. Verfahren nach einem oder mehreren der vorangehenden Ansprüche, dadurch gekennzeichnet, daß in die Reaktionsbehälter zusammen mit dem Wasser geeignete Substrate gegeben werden, die das Wachstum der Mikroorganismen im Biokatalysator fördern und/oder einen Abbau der persistenten organischen Inhaltsstoffe durch einen Co-Metabolismus ermöglichen.

14. Verfahren nach einem oder mehreren der vorangehenden Ansprüche, dadurch gekennzeichnet, daß im Reaktionsbehälter, der die Biokatalysatoren enthält, in die Mikroorganismen so eingeschlossen sind, daß sie das Polymer nicht durchdringen und gleichzeitig Fremdkeime oder Protozoen nicht in die Gelkugeln eindringen können, das Wachstum konkurrierender Mikroorganismen außerhalb des Biokatalysators unterdrückt wird, indem das Medium umgepumpt und mittels geeigneter Vorrichtungen von Fremdkeimen weitgehend entkeimt wird.

Europäisches
Patentamt

**EUROPÄISCHER RECHERCHENBERICHT**

Nummer der Anmeldung

EP 91 25 0197
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 320 483 (MONSANTO COMPANY)<br>* Spalte 11; Ansprüche 1-3,6,10,11,12,14,29 *<br>* Spalte 2, Zeile 10 - Zeile 23 *<br>* Spalte 2, Zeile 33 - Zeile 47 *<br>* Spalte 4, Zeile 30 - Spalte 5, Zeile 15 *<br>* Spalte 5, Zeile 41 - Zeile 48 *<br>* Spalte 5, Zeile 58 - Spalte 6, Zeile 16 *<br>* Spalte 6, Zeile 40 - Zeile 42 *<br>--- | 1,4,7 | C02F3/10<br>C12N11/04<br>C02F3/06<br>C02F3/34 |
| A | PATENT ABSTRACTS OF JAPAN<br>vol. 10, no. 335 (C-384)(2391) 13. November 1986<br>& JP-A-61 139 385 ( SUSUMU HASHIMOTO ) 26. Juni 1986<br>* Zusammenfassung *<br>--- | 1 | |
| A | WO-A-8 808 825 (SCHERING AG)<br><br>* Seite 16; Ansprüche 1-5,7,8 *<br>* Seite 1, Absatz 1 *<br>* Seite 1, Absatz 5 *<br>* Seite 2, Absatz 5 - Seite 3, Absatz 1 *<br>* Seite 4, Absatz 2 - Seite 5, Absatz 1 *<br>Beispiel 6<br>* Seite 11 *<br>--- | 1,4,6-8, 12 | |
| A | US-A-4 755 296 (OCCIDENTAL CHEMICAL CORPORATION)<br>* Spalte 10; Anspruch 1 *<br>* Spalte 6; Tabelle 1 *<br>--- | 1,2,9,10 | |
| A | US-A-4 857 198 (ZIMPRO/PASSAVANT)<br>* Spalte 6; Anspruch 1 *<br>--- | 1,11 | |
| A | FR-A-2 337 108 (KURARAY)<br>* Seite 15; Ansprüche 1-5 *<br>--- | 14 | |

RECHERCHIERTE SACHGEBIETE (Int. Cl.5)

CO2F
C12N

-/--

Der vorliegende Recherchenbericht wurde für alle Patentansprüche erstellt

| Recherchenort | Abschlußdatum der Recherche | Prüfer |
|---|---|---|
| DEN HAAG | 25 NOVEMBER 1991 | TEPLY J. |

Europäisches
Patentamt

**EUROPÄISCHER RECHERCHENBERICHT**

Nummer der Anmeldung

EP    91 25 0197
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A | BIOTECHNOLOGY AND BIOENGINEERING. Bd. 38, Nr. 3, Juli 1991, NEW YORK US Seiten 273 - 279; JIAN-ER LIN ET AL.: 'USE OF COIMMOBILIZED BIOLOGICAL SYSTEMS TO DEGRADE TOXIC ORGANIC COMPOUNDS' * Seite 273 - Seite 274, Absatz 2 * * Seite 274, linke Spalte, letzter Absatz - rechte Spalte, Absatz 1 * * Seite 277, rechte Spalte, letzter Absatz - Seite 278, linke Spalte, Absatz 2 * ----- | 1,4,6,8, 14 | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| DEN HAAG | 25 NOVEMBER 1991 | TEPLY J. |